# EUROPÄISCHE PATENTSCHRIFT

(11) **EP 2 016 030 B1**
(45) Veröffentlichungstag und Bekanntmachung des Hinweises auf die Patenterteilung: **10.04.2013**
(21) Anmeldenummer: 07724856.5
(22) Anmeldetag: 04.05.2007
(51) Int. Cl.: C02F 3/28

(54) **REAKTOR ZUR ANAEROBEN REINIGUNG VON ABWASSER**
REACTOR FOR THE ANAEROBIC CLEANING OF WASTE WATER
RÉACTEUR POUR L'ÉPURATION ANAÉROBIE D'EAUX USÉES

(30) Priorität: 04.05.2006 DE 102006020709
(43) Veröffentlichungstag der Anmeldung: 21.01.2009
(73) Patentinhaber: Enviro-Chemie GmbH, 64380 Rossdorf (DE)
(72) Erfinder: GÖTZ, Willi, 64404 Bickenbach (DE); HELLMANN, Werner, 50226 Frechen (DE)
(74) Vertreter: Behrens, Helmut
(86) Internationale Anmeldenummer: PCT/EP2007/003931
(87) Internationale Veröffentlichungsnummer: WO 2007/128503

(56) Entgegenhaltungen:
- EP-A1- 1 408 008
- GB-A- 1 535 591
- US-A- 5 698 102

## Beschreibung

Die Erfindung betrifft einen Reaktor zur anaeroben Reinigung von Abwasser.

Derartige Reaktoren sind in unterschiedlichen Ausführungsformen bekannt, wobei ein Reaktortyp als UASB-Reaktor (Upflow-Anaerobic-Sludge-Blanket-Reactor) bezeichnet wird. Dieser Reaktortyp ist unter anderem aus dem Buch "Anaerobtechnik Handbuch der anaeroben Behandlung von Abwasser und Schlamm" Springer Verlag, Prof. Dr.-Ing. Dr.h.c. Dr. E.h. B. Böhnke, Prof. Dr.-Ing. W. Bischofsberger, Prof. Dr.-Ing. C.F. Seyfried, Kap. 6.4.1, vorbekannt. Bei diesem UASB-Reaktor, dem ein biologisches Verfahren zugrunde liegt, wird die Fähigkeit von biologischen Bakterien ausgenutzt, organische Substanzen abzubauen und in niedermolekulare Substanzen umzusetzen. Bei diesen Umsetzungsprozessen entsteht Biomasseschlamm, Biogas sowie das gereinigte Wasser.

Ein Beispiel aus der Patentliteratur ist EP 1408008.

Der UASB-Reaktor besteht im wesentlichen aus einem geschlossenen Behälter, der von unten nach oben mit dem zu reinigenden Abwasser durchströmt wird, wobei sich im unteren Bereich des Behälters eine mit Mikroorganismen (Bakterien) versetzte Schlammzone, das sogenannte Schlammbett, befindet. Im, Kopfbereich des Reaktors ist eine sogenannte Dreiphasen-Trenneinrichtung angeordnet, die eine Abtrennung von Gas, dem gereinigten Abwasser und Schlammpartikeln bewirkt. Um eine gute Reinigungsleistung des Reaktors zu erzielen und einen kontinuierlichen störungsfreien Betrieb des Reaktors sicherzustellen, wird insbesondere die gleichmäßige Verteilung des zugeführten zu reinigenden Abwassers als auch eine sichere Trennung von Gas, Wasser und Schlammpartikeln in der Dreiphasen-Trenneinrichtung benötigt.

Der Erfindung liegt daher die Aufgabe zugrunde, einen UASB-Reaktor derart weiterzubilden, dass eine gute Reinigungsleistung sowie ein kontinuierlicher störungsfreier Betrieb und ein geringer Wartungsaufwand des Reaktors erzielt wird.

Diese Aufgabe wird durch die im Patentanspruch 1 angegebenen Merkmale gelöst.

Nachfolgend wird ein Ausführungsbeispiel des erfindungsgemäßen Reaktors unter Bezugnahme auf die Figuren erläutert.

Es zeigen:
- Fig. 1:: eine Darstellung des Zulaufrohrsystems im Bereich des Behälterbodens in Draufsicht; und
- Fig. 2:: eine vergrößerte Darstellung eines Querschnittes durch ein Abscheidepaket.

Ein Reaktor zur anaeroben Abwasserbehandlung wird in der industriellen Abwasserbehandlung eingesetzt.

Der Reaktor besteht dabei im wesentlichen aus einem geschlossenen im Querschnitt rechteckigen Behälterraum 2, einem Zulaufrohrsystem 3 für das zu behandelnde Abwasser im Bodenbereich des Behälterraums 2, einer Dreiphasen-Trenneinrichtung im Kopfbereich des Behälterraums 2 sowie einer im oberen Endbereich mittig angeordneten horizontal längsverlaufenden Wasserablaufrinne und ebenfalls im oberen Endbereich angeordneten Gassammelbereichen sogenannten Gasdomen mit Gasabführleitungen.

Im Bodenbereich des Behälterraums 2 befindet sich eine mit Mikroorganismen versetzte Aktivschlammzone, in die eine Vielzahl von Zuführleitungen für das zu behandelnde Abwasser mündet. Die konstruktive Ausgestaltung des Zulaufrohrsystems 3 wird nachfolgend näher erläutert.

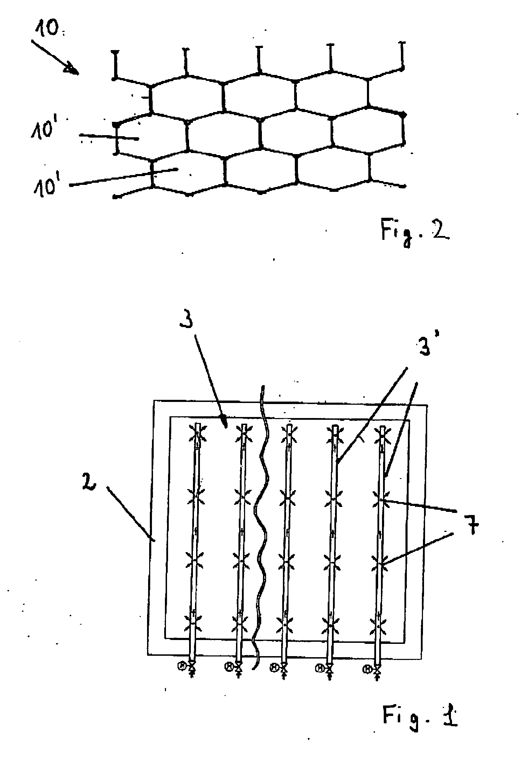

Wie es aus der Schnittdarstellung zur Fig. 1 zu ersehen ist, besteht das Zulaufrohrsystem 3 aus einer Vielzahl parallel zueinander ausgerichteter Rohrleitungen 3', die sich im unteren Endbereich des Behälterraums 2 in einer Höhe von ca. 100 - 250mm horizontal beabstandet vom Behälterboden über die gesamte Breite des Behälterraums 2 erstrecken. Die Rohrleitungen 3' besitzen jeweils regelmäßig beabstandet angeordnete Einströmdüsen 7, wobei das Einströmen des zu behandelnden Abwassers in Fig. 1 durch die Pfeile dargestellt ist. Diese Rohrleitungen sind mit einem entsprechenden Mess-Regelsystem ausgestattet, so dass zum einen der Zulauf der einzelnen Rohrleitungen 3' individuell geregelt und zum anderen Störungen in Folge von Verstopfungen sofort erkannt und beseitigt werden können. Die Rohrleitungen 3' sind mit einem Spülanschluss versehen, so dass nach Bedarf die Rohrleitungen 3' gespült werden können.

Wie bereits zuvor beschrieben, erfolgt die Anströmung des Reaktors über das Zulaufrohrsystem 3 gleichmäßig über die gesamte Grundfläche des Reaktors. Durch diese Zuführung des Abwassers und geeignete Steuerung der Anströmgeschwindigkeit verbleibt die aktive Biomasse überwiegend im Höhenbereich bis maximal 2,5 m Reaktorhöhe über dem Behälterboden. In diesem Bereich findet auch der wesentliche anaerobe Umsatz der organischen Inhaltsstoffe in Gas statt.

Ein Teil der dabei gebildeten Gasblasen agglomerieren im Reaktor zu immer größer werdenden Gasblasen, steigen auf und trennen sich in den im oberen Endbereich jeweils an den Längsseiten des Behälterraumes 2 angeordneten Gassammelbereichen von Wasser ab. Die Gassammelbereiche sind jeweils mit Gasabführleitungen versehen und führen das Biogas ab.

Durch die Strömungsführung gelangt das mit leichter Biomasse gering beladene Abwasser in den oberen Behälterraum.

Im oberen Bereich des Behälterraums 2 in dessen Kopfbereich, ist mittig eine sogenannte Dreiphasen-Trennvorrichtung angeordnet, in der eine Abtrennung von Restgas, Biomasseschlamm und gereinigtem Wasser erfolgt.

Die Dreiphasen-Trenneinrichtung ist an einer mittig im Behälterraum 2 angeordneten Stützkonstruktion befestigt und besteht im wesentlichen aus zwei parallel nebeneinander in Längsrichtung des Reaktors angeordneten V-förmig ausgebildeten Rinnen, in die jeweils ein sogenanntes Abscheidepaket 10 eingefügt ist.

Die Dreiphasen-Trenneinrichtung bzw. die v-förmigen Rinnenkonstruktionen sind derart ausgestaltet, dass das Gas-Wasser-Schlamm-Gemisch durch die eingebaute Konstruktion entweder über die rechts oder linksseitig angeordneten Gassammelbereiche geleitet werden und anschließend durch eingebaute Schikanen das Wasser-Schlamm-Gemisch nach unten umgelenkt und dem Abscheidepaket 10 zugeführt wird. Durch die Umlenkung der Wasserströmung erhält man eine Tauchung, wodurch ein Gasdurchtritt in den Bereich der Dreiphasen-Trenneinrichtung verhindert wird.

Die Biomassen-Wasser-Trennung erfolgt hierbei in zwei parallel eingebauten Abscheidepakten 10, welche in Längsrichtung horizontal verlaufend im Reaktor eingebaut sind. Die Abscheidepakete 10 liegen jeweils mit ihrer einander zugewandten Seitenwand an der der Behältermitte zugewandten Seitenwand der v-förmig ausgebildeten Rinne an. Hierdurch erhält man eine schräggestellte Anordnung der Abscheidepakete 10, die in einem Winkel von 15° bis 45° variieren kann. Nähere konstruktive Einzelheiten sind der Schnittdarstellung in Fig. 2 zu entnehmen. Aus Fig. 2 ist ersichtlich, dass das Abscheidepaket aus einer Vielzahl nebeneinanderliegend angeordneter röhrenförmiger Abschnitte 10' besteht, wobei diese im Querschnitt eine Wabenstruktur besitzen. Im dargestellten Ausführungsbeispiel weist die Wabenstruktur ein Sechseckprofil auf. Die röhrenförmige Abschnitte können auch einen anderen Querschnitt beispielsweise einen kreisförmigen Querschnitt besitzen. Die Abscheidepaktete 10 sind vorzugsweise aus Kunststoffen hergestellt.

Wie bereits zuvor kurz beschrieben, wird das Wasser-Schlamm-Gemisch über die Gassammelräume durch eingebaute Schikanen in der Richtung umgekehrt und komplett in der Durchflussrichtung von unten nach oben durch die schräg eingebauten Abscheidepakete 10 zu einer Ablaufrinne geführt. Durch den Schrägeinbau der Abscheidepakete 10 ist sichergestellt, dass das gesamte Wasser-Schlamm-Gemisch durch die Abscheidepakete 10 geführt wird. Aufgrund der konstruktiven Ausgestaltung der Abscheidepakete 10 und des oben genannten Einbaus wird eine optimal große Abscheidefläche erreicht, wodurch eine effiziente Biomassen-Wasser-Trennung erzielt werden kann. Aufgrund des Schrägeinbaus der Abscheidepakete 10 und der Wabenstruktur im Querschnitt wird eine gesamte horizontal projizierte Oberfläche von ca. 25-60 m²/lfd. Meter Reaktorlänge erzielt.

Mittig im oberen Bereich der Dreiphasen-Trenneinrichtung ist eine horizontal längs verlaufende Wasserablaufrinne angeordnet, die im wesentlichen einen rechteckigen Querschnitt besitzt und wobei die in Längsrichtung verlaufenden oberen Kanten ein Zackenprofil aufweisen. Das durch die Abscheidepakete 10 geführte vom Biomassenschlamm getrennte Wasser kann nachfolgend über die gezackten Einlaufkanten in die Wasserablaufrinne strömen und wird abgeführt. Die Wasserablaufrinne ist höhenverstellbar ausgebildet. Durch die Ausbildung der Wasserablaufrinne werden stark beruhigte Ablaufgeschwindigkeiten erreicht, wodurch ein mitziehen von Feinstpartikeln verhindert wird. Eventuell gebildeter Schwimmschlamm wird über eine beidseitig an der Reaktorstirnseite angebrachte nicht dargestellten Überlauf abgezogen.

Der in den Abscheidepaketen 10 abgetrennte Schlamm wird aufgrund der konstruktiven Ausgestaltung der v-förmigen Rinnen nach unten geleitet und gelangt über eine im unteren Bereich der v-förmigen Rinnen angeordnete Austrittsöffnung in den Behälterraum 2 zurück. Bedingt durch die im Wasser-Schlamm-Raum erhöhte Dichte des Wassers und durch den zurücklaufenden Schlamm wird innerhalb des Abscheidepaketes 10 eine Gassperre aufgebaut, welche einen Gasdurchtritt von unten aus dem Behälterraum 2 in den Abscheideraum der Dreiphasen-Trenneinrichtung und damit in die Atmosphäre verhindert. Weiterhin ist im unteren Bereich der v-förmigen Rinnen jeweils eine Reinigungseinrichtung vorgesehen, die eine Ablagerung von Schlammbrücken in dem Durchtrittsbereich zum Behälterraum 2 entfernt. Die Reinigungsvorrichtung ist hierzu als Schlammscraper ausgebildet, der angepasst an das Rinnenprofil einen dreieckigen Querschnitt aufweist und über eine Seilkonstruktion periodisch in Längsrichtung am Bodenbereich der v-förmigen Rinnen entlang gezogen werden kann.

Im Bereich des Zu- und Ablaufes des Reaktors ist weiterhin eine Messeinrichtung zur elektrooptische Trübungsmessung zwecks Online-Überwachung der Wasserqualität hinsichtlich der Trübstoffe installierbar. Mit dieser Messeinrichtung wird die jeweilige Konzentration an partikulären und emulgierten Trübstoffen wie z.B. Bioschlamm, emulgierten Fetten oder feinpartikulären Lebensmittelresten durch den Einfluss auf die Lichtstreuung erfasst. Die Messsignale der Trübungsmessung werden in dem Mess-Regelsystem ausgewertet und der Zulaufvolumenstroms entsprechend der anlagenspezifischen Kennlinie geregelt. Durch diese Überwachungs- und Mess-Regelungstechnik wird eine hohe Ablaufqualität mit geringer Trübstoffbelastung erreicht und der Austrag von Bioschlamm vermieden. Die Trübungsmessung ermöglicht neben der Onlineregelung auch eine Fernüberwachung der Reinigungsleistung des Reaktors. Die entsprechende Messeinrichtung und das Mess-Regelsystem ist zeichnerisch nicht dargestellt.

## Patentansprüche

1. Reaktor zum anaeroben biologischen Reinigen von Abwasser, umfassend einen geschossenen Behälter, der im Bodenbereich ein Zulaufrohrsystem für das Abwasser und im Kopfbereich eine Ablaufeinrichtung für das gereinigte Wasser aufweist, und wobei zwischen Zulaufrohrsystem und Ablaufeinrichtung eine Dreiphasen-Trenneinrichtung und mindestens ein Gas-Sammelraum angeordnet ist, wobei die Dreiphasen-Trenneinrichtung mindestens eine nach unten sich verjüngende Rinne umfasst und wobei mindestens ein Abscheidepaket in der Rinne angeordnet ist und an der Rinne Schikanen angeordnet sind, so dass mindestens das zu trennende Wasser-Schlammgemisch derart umgelenkt wird, dass es das Abscheidepaket durchströmt und wobei unterhalb des mindestens einen Abscheidepaktetes im Bodenbereich der Rinne ein Schlammabsetzbereich mit Durchtrittsöffnung gebildet wird, so dass ein Absinken der abgetrennten Schlammpartikel in den unteren Behälterraum ermöglicht wird, **dadurch gekennzeichnet, dass** das Zulaufrohrsystem (3) aus einer Vielzahl von einzelnen Rohrleitungen (3') mit Einströmdüsen (7) besteht, die über ein Mess-Regelsystem einzeln ansteuerbar ausgebildet sind, wobei die Dreiphasen-Trennvorrichtung aus zwei nebeneinander angeordneten sich nach unten verjüngenden Rinnen besteht und in jeder Rinne ein in Rinnenlängsrichtung sich erstreckendes Abscheidepaket (10) angeordnet ist, und dass jedes Abscheidepaket (10) aus einer Vielzahl nebeneinanderliegend angeordneter röhrenförmiger Abschnitte (10') besteht, und dass jedes Abscheidepaket (10) schräg in die Rinne der Dreiphasen-Trenneinrichtung eingebaut ist, so dass das zu trennende Wasser-Schlamm-Gemisch von unten nach oben durch die das Abscheidepaket (10) bildenden Einzelröhren (10') strömt und die Strömungsrichtung unter einem Winkel zwischen 15° und 45° zur Vertikalachse des Reaktors erfolgt, wobei an den Längsseiten des Behälterraums (2) in dessen Kopfbereich jeweils benachbart der Rinnen Gas-Sammelräume angeordnet sind, und dass das Gas-Wasser-Schlammgemisch zunächst über die Gas-Sammel-Räume und anschließend das Wasser-Schlammgemisch über Schikanen umgelenkt in die Dreiphasen-Trenneinrichtung geführt wird, und dass im Bodenbereich der Rinne eine Reinigungseinrichtung zur Beseitigung von Schlammablagerungen angeordnet ist, die als Schlammscraper ausgebildet ist.

2. Reaktor zum anaeroben biologischen Reinigen von Abwasser nach Patentanspruch 1, wobei die röhrenförmigen Abschnitte (10') im Strömungsquerschnitt eine Wabenstruktur besitzen.

3. Reaktor zum anaeroben biologischen Reinigen von Abwasser nach Patentanspruch 1 oder 2, wobei das Zulaufrohrsystem (3) aus einer Vielzahl parallel zueinander ausgerichteter Rohrleitungen (3') besteht.

4. Reaktor zum anaeroben biologischen Reinigen von Abwasser, nach einem der vorhergehenden Patentansprüche wobei die Abscheidepakete (10) aus Kunststoff ausgeführt sind.

5. Reaktor zum anaeroben biologischen Reinigen von Abwasser nach einem der vorhergehenden Patentansprüche, einen dreieckigen Querschnitt aufweist, der dem Rinnenprofil angepasst ist und wobei der Schlammscaper mittels einer Seilkonstruktion längs des Bodenbereiches der Rinnen entlang gezogen werden kann.

6. Reaktor zum anaeroben biologischen Reinigen von Abwasser nach einem der vorhergehenden Patentansprüche, wobei die Wasserablaufrinne für das gereinigte Wasser mittig zwischen den Rinnen der Dreiphasen-Trennvorrichtung angeordnet und als Zackenwehrrinne ausgebildet ist.

7. Reaktor zum anaeroben biologischen Reinigen von Abwasser nach Patentanspruch 6, wobei die Zackenwehrrinne höhenverstellbar angeordnet ist.

8. Reaktor zum anaeroben biologischen Reinigen von Abwasser nach einem der vorhergehenden Patentansprüche, wobei im Bereich des Zu- und des Ablaufs des Reaktors eine Messeinrichtung zur elektrooptischen Trübungsmessung vorgesehen ist, und die ermittelten Messsignale dem Mess-Regelsystem zugeführt werden und anhand einer im Mess-Regelsystem abgelegten Kennlinie ein Ist/Soll-Vergleich stattfindet und das Ausgangssignal dieser Auswertung den Zulaufvolumenstrom regelt.

## Claims

1. Reactor for the anaerobic biological cleaning of waste water, comprising a closed tank, which has a feed pipe system for the waste water in the base region and a discharge device for the cleaned water in the head region, and wherein a three-phase separating device and at least one gas collecting space are arranged between the feed pipe system and discharge device, wherein the three-phase separating device comprises at least one downwardly tapering channel and wherein at least one separating assembly is arranged in the channel and baffles are arranged on the channel, so that at least the water/sludge mixture to be separated is deflected in such a way that it flows through the separating assembly and wherein a sludge depositing region with a through-opening is formed below the at least one separating assembly in the base region of the channel, so that a sinking of the separated sludge particles into the lower tank space is made possible, **characterised in that** the feed pipe system (3) consists of a multiplicity of individual pipelines (3') with inflow nozzles (7), which can be activated individually by means of a measuring control system, the three-phase separating system consisting of two channels tapering downwardly and arranged next to one another and a separating assembly (10) arranged in each channel extending in the longitudinal direction of the channel, and **in that** each separating assembly (10) consists of a multiplicity of tubular portions (10') arranged lying next to one another, and **in that** each separating assembly (10) is installed obliquely into the channel of the three-phase separating device, so that the water/sludge mixture to be separated flows from bottom to top through the individual pipes (10') forming the separating assembly (10) and the flow direction is at an angle of between 15° and 45° to the vertical axis of the reactor, gas collecting spaces being arranged on the longitudinal sides of the tank space (2) in the head region thereof in each case adjacent to the channels, and **in that** the gas/water/sludge mixture is firstly deflected by way of the gas collecting spaces and subsequently the water/sludge mixture is deflected by way of baffles into the three-phase separating device, and **in that** a cleaning device for removing sludge deposits is arranged in the base region of the channel and is configured as a sludge scraper.

2. Reactor for the anaerobic biological cleaning of waste water according to claim 1, wherein the tube-shaped portions (10') have a honeycomb structure in the flow cross section.

3. Reactor for the anaerobic biological cleaning of waste water according to claim 1 or 2, wherein the feed pipe system (3) consists of a plurality of pipelines (3') oriented parallel to one another.

4. Reactor for the anaerobic biological cleaning of waste water according to any one of the preceding claims, wherein the separating assemblies (10) are made of plastics material.

5. Reactor for the anaerobic biological cleaning of waste water according to any one of the preceding claims, which has a triangular cross section, which is adapted to the channel profile and wherein the sludge scraper can be drawn by means of a cable construction along the base region of the channels.

6. Reactor for the anaerobic biological cleaning of waste water according to any one of the preceding claims, wherein the water discharge channel for the cleaned water is arranged centrally between the channels of the three-phase separating device and is configured as a toothed weir channel.

7. Reactor for the anaerobic biological cleaning of waste water according to claim 6, wherein the toothed weir channel is height-adjustably arranged.

8. Reactor for the anaerobic biological cleaning of waste water according to any one of the preceding claims, wherein a measuring device for the electro-optical measurement of turbidity is provided in the region of the feed and discharge of the reactor, and the measuring signals determined are fed to the measuring control system and an actual/desired comparison takes place using a characteristic stored in the measuring control system and the output signal of this evaluation controls the feed volume flow.

## Revendications

1. Réacteur pour l'épuration biologique anaérobie d'eaux usées, comprenant un récipient fermé qui comporte dans la zone du fond un système de tuyaux d'amenée pour l'eau usée et dans la zone de tête une conduite de sortie pour l'eau épurée, un dispositif de séparation à trois phases et au moins un compartiment collecteur de gaz étant disposés entre le système de tuyaux d'amenée et la conduite de sortie, le dispositif de séparation à trois phases comportant au moins une rigole se rétrécissant vers le bas et au moins un pack de séparation étant disposé dans la rigole, et des chicanes étant disposées sur la rigole, de telle sorte qu'au moins le mélange d'eau et de boue à séparer soit dévié de telle sorte qu'il traverse le pack de séparation et en dessous dudit au moins un pack de séparation, une zone de dépôt de la boue étant formée dans la région du fond de la rigole avec des orifices de passage, de telle sorte qu'une sédimentation des particules de boue séparées dans l'espace inférieur du récipient est rendue possible, **caractérisé en ce que** le système de tuyaux d'amenée (3) consiste en une multitude de conduites tubulaires individuelles (3') avec des gicleurs d'introduction (7) qui sont configurés pour être adressables individuellement par l'intermédiaire d'un système de mesure et de régulation, le dispositif de séparation à trois phases consistant en deux rigoles disposées l'une à côté de l'autre, se rétrécissant vers le bas, tandis qu'un pack de séparation (10), s'étendant dans la direction longitudinale de la rigole, est disposé dans chaque rigole, et que chaque pack de séparation (10) consiste en une multitude de tronçons tubulaires (10') disposés les uns à côté des autres, et que chaque pack de séparation (10) est monté obliquement dans la rigole du dispositif de séparation à trois phases, de telle sorte que le mélange d'eau et de boue à séparer s'écoule de bas en haut à travers les tuyaux individuels (10') formant le pack de séparation (10), l'écoulement s'effectuant dans une direction sous un angle compris entre 15° et 45° par rapport à l'axe vertical, des espaces collecteurs de gaz étant disposés sur les côtés longitudinaux de l'espace du récipient (2), dans sa zone de tête, respectivement au voisinage des rigoles, et le mélange gaz-eau-boue étant d'abord conduit dans le dispositif de séparation à trois phases par les espaces collecteurs de gaz, et ensuite le mélange eau-boue étant dévié par des chicanes, et un dispositif de nettoyage configuré comme grattoir de boue étant disposé dans la zone du fond de la rigole pour l'élimination des dépôts de boue.

2. Réacteur pour l'épuration biologique anaérobie d'eaux usées selon la revendication 1, dans lequel les tronçons en forme de tubes (10') possèdent dans la section de l'écoulement une structure en nid d'abeilles.

3. Réacteur pour l'épuration biologique anaérobie d'eaux usées selon la revendication 1 ou 2, dans lequel le système de tuyaux d'amenée (3) consiste en une multitude de tuyauteries (3') orientées parallèlement les unes aux autres.

4. Réacteur pour l'épuration biologique anaérobie d'eaux usées selon l'une quelconque des revendications précédentes, dans lequel les pack séparateurs (10) sont réalisés en plastique.

5. Réacteur pour l'épuration biologique anaérobie d'eaux usées selon l'une quelconque des revendications précédentes, présentant une section triangulaire qui est adaptée au profil de la rigole, et le grattoir à boue pouvant être traîné le long de la zone du fond des rigoles au moyen d'une construction à câble.

6. Réacteur pour l'épuration biologique anaérobie d'eaux usées selon l'une quelconque des revendications précédentes, dans lequel la rigole d'évacuation d'eau pour l'eau épurée est disposée au milieu entre les rigoles du dispositif séparateur à trois phases et est configurée comme rigole de déversoir à dents.

7. Réacteur pour l'épuration biologique anaérobie d'eaux usées selon la revendication 6, dans lequel la rigole de déversoir à dents est disposée de façon à être réglable en hauteur.

8. Réacteur pour l'épuration biologique anaérobie d'eaux usées selon l'une quelconque des revendications précédentes, dans lequel, dans les zones d'entrée et de sortie du réacteur, un dispositif de mesure est prévu pour la mesure électro-optique de la turbidité, et dans lequel les signaux de mesure déterminés sont acheminés au système de mesure et de régulation et dans lequel, au vu d'une courbe caractéristique enregistrée dans le système de mesure et de régulation, il s'effectue une comparaison réel/consigne, le signal de sortie de cette évaluation régulant le débit volumique entrant.
